# EUROPÄISCHE PATENTSCHRIFT

(11) **EP 0 553 596 B1**
(45) Veröffentlichungstag und Bekanntmachung des Hinweises auf die Patenterteilung: **29.03.1995**
(21) Anmeldenummer: 92810941.2
(22) Anmeldetag: 02.12.1992
(51) Int. Cl.: A61F 2/42

(54) **Fingergelenkprothese aus Metall**
Metallic finger joint prosthesis
Prothèse métallique pour articulation du doigt

(30) Priorität: 31.01.1992 CH 286/92
(43) Veröffentlichungstag der Anmeldung: 04.08.1993
(73) Patentinhaber: SULZER Medizinaltechnik AG, 8404 Winterthur (CH)
(72) Erfinder: Koch, Rudolf, CH-8500 Frauenfeld (CH); Sandoz, Yvan, CH-8405 Winterthur (CH)
(74) Vertreter: Hammer, Bruno, Dr.

(56) Entgegenhaltungen:
- DE-A- 2 814 752
- US-A- 3 816 854
- US-A- 3 990 118
- US-A- 4 293 963

## Beschreibung

Die Erfindung handelt von einer Fingergelenkprothese aus Metall, die aus einer Lagerschale besteht, die mit einem Schaft im Metakarpal verankert ist und die einen Lagerzapfen führt, welcher mit einem Schaft im Phalanx verankert ist, wobei der Lagerzapfen als Tonne ausgeführt ist, die quer zu ihrer Achse in ihrer Mitte ein Verbindungsstück zu ihrem Schaft aufweist.

Fingergelenkprothesen verlangen bei ihrer Implantation dem Operateur eine enorme Geschicklichkeit ab, da Finger im Gelenkbereich ausserhalb des Knochengewebes nur einige wenige Blutgefässe, die für die Bewegung notwendigen Bänder, für den Tastsinn notwendige Nerven, sowie umhüllende Haut aufweisen. Um die Versorgung des äusseren Fingerteils nicht zu gefährden sollten die Teile ausserhalb des Knochengewebes in ihrer Funktion gegen proximal möglichst wenig beeinträchtigt werden. Gleichzeitig sollte der Gelenkersatz den natürlichen Bewegungsbereich möglichst gut nachbilden.

In der Patentschrift US 4,293,963 wird ein künstliches Ellbogengelenk beschrieben, bei dem eine begrenzte medio-laterale Schwenkung und eine Beuge- und Streckbewegung erreicht werden, wobei extreme medio-laterale Schwenkungen der Achse eines metallischen Lagerzapfens durch Seitenwände an einer Lagerschale aus Polyäthylen begrenzt werden. Die Lagerschale ist dabei soweit zurückgeschnitten, dass der Lagerzapfen radial ein- und ausgefahren werden kann. Die gezeigte Art der Lagerung liesse sich auf eine Fingergelenkprothese abwandeln, wobei sie einmal ein grosses Volumen beansprucht und zum anderen der Gelenkzapfen in der Gelenkkapsel in radialer Richtung nicht gefangen ist.

Der Oberbegriff des Patentanspruchs 1 geht von einer Fingergelenkprothese gemäß US-A-3990118 aus.

Hier schafft die Erfindung Abhilfe. Sie hat die Aufgabe eine Fingergelenkprothese zu schaffen, bei der der Gelenkzapfen in radialer Richtung gefangen wird, und die intraoperativ das radiale Einsetzen des Gelenkzapfens in die Lagerschale ermöglicht. Diese Aufgabe wird mit den kennzeichnenden Merkmalen vom Hauptanspruch 1 gelöst.

Die Vorteile der Erfindung sind darin zu sehen, dass mit einem Minimum an Teilen ein gefangenes Gelenk erzeugt wird, das sich intraoperativ in abgewinkelter Stellung nach der definitiven Verankerung des Lagerzapfens im Phalanx zusammenstecken lässt. Auf diese Weise werden die im Bereich des Gelenks liegenden Blutgefässe und Gewebeteile nicht überbeansprucht, da sie bei einem Längsschnitt durch die Haut der Oberseite in abgewinkelter Stellung vom Knochen weg seitlich ausgelenkt werden können. Die abhängigen Ansprüche 2 bis 6 stellen vorteilhafte Weiterbildungen der Erfindung dar.

Im folgenden wird die Erfindung anhand von einem Ausführungsbeispiel beschrieben. Es zeigen:

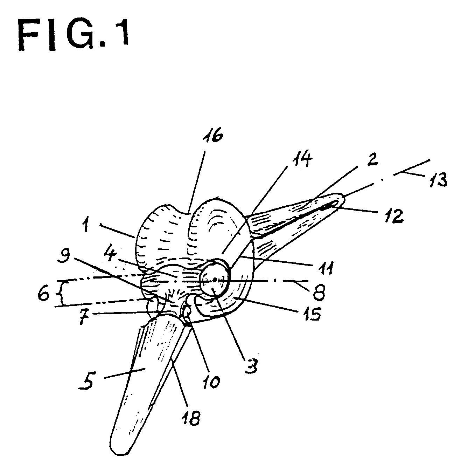

- Figur 1: Die Ansicht einer radial zusammengesteckten Fingergelenkprothese, bei der eine Lagerschale den Lagerzapfen C-förmig umschliesst;
- Figur 2: die Seitenansicht einer Fingergelenkprothese nach Fig. 1 in annähernd gestreckter Stellung, und
- Figur 3: die Seitenansicht einer Fingergelenkprothese nach Fig. 1 in abgewinkelter Stellung, bei der die Lagerschale durch den radial einfahrenden Lagerzapfen aufgespreizt ist.

In den Figuren ist eine Fingergelenkprothese aus Metall gezeigt, die aus einer Lagerschale 1 besteht, die mit einem Schaft 2 im Metakarpal verankert ist und die einen Lagerzapfen 3 führt, welcher mit einem Schaft 5 im Phalanx verankert ist. Der Lagerzapfen 3 ist als Tonne 4 ausgeführt, die quer zu ihrer Achse 8 in ihrer Mitte ein Verbindungsstück 7 zu ihrem Schaft 5 aufweist. Die Lagerschale 1 umschliesst die Tonne 4 C-förmig mit Spiel, welches die Seitenauslenkung des Phalanx Schaftes 5 begrenzt, während ein Durchbruch 6 im "C" die Flexion am Verbindungsstück 7 begrenzt. Die C-förmige Lagerschale 1 ist beim Einsetzen der Tonne 4 aufspreizbar, dadurch dass sie mit ihrem Schaft 2 durch einen schlitzförmigen Einschnitt 11 in Längsrichtung 13 in zwei Hälften 14, 15 geteilt ist.

Der als Tonne 4 ausgebildete Lagerzapfen 3 ist über ein Verbindungsstück 7 mit seinem Schaft 5 verbunden, welcher sich konisch verjüngt und eine texturierte Oberfläche zur zementfreien Verankerung im Phalanx aufweist. Die Primärverankerung wird zusätzlich durch Längsflossen 18 unterstützt, welche sich im vorbereiteten Knochenhohlraum eingraben und primär der Rotationssicherung dienen. Nach der Resektion von Phalanx und Metakarpal (hier nicht gezeigt) werden die Knochenhohlräume für die Aufnahme der Schäfte 2, 5 in abgewinkelter Stellung mit konischen Reibahlen vorbearbeitet. Anschliessend wird der Lagerzapfen 3 mit seinem Schaft 5 definitiv im Phalanx verankert. Die Lagerschale 1 besitzt ebenfalls einen sich konisch verjüngenden Schaft 2 mit Längsflossen 18. Sie ist C-förmig ausgebildet, um die Tonne 4 mit Spiel zu umschliessen. Der Durchbruch 6 in der C-Form begrenzt einen Flexionsbereich 17, indem das als Hals 9 ausgeführte Verbindungstück 7 am oberen oder unteren Lagerrand anschlägt, wobei der untere Lagerrand eine Ausnehmung 10 aufweist, in die der Hals 9 eintaucht. Auf diese Weise entsteht im abgewinkelten Zustand eine engere Seitenführung und eine Vergrösserung des Flexionsbereiches 17. Die Lagerschale 1 und ihr zugehöriger Schaft 2 weisen in der Längsrichtung 13 einen von der Lagerschale 1 ausgehenden schlitzförmigen Einschnitt 11 auf, der die Lagerschale 1 und ihren Schaft 2 in zwei Hälften 14, 15 teilt. Dieser Schlitz wird vorzugsweise mit einer Geraden als Erzeugenden, die parallel zur Achse 8 ausgerichtet ist, durch z.B. funkenerosives Drahterodieren erzeugt und endet in einem Querdurchbruch 12 vor dem Ende von Schaft 2, damit Lagerschale 1 und ihr Schaft 2 einstückig bleiben und trotzdem zum radialen Einbringen vom Lagerzapfen 3 aufspreizbar sind. Der Schaft 2 wirkt bei dieser Ausführung wie eine gekrümmte Blattfeder, welche die Lage der beiden Hälften 14, 15 zueinander bestimmt und ein Aufspreizen gegen eine Schliesskraft möglich macht.

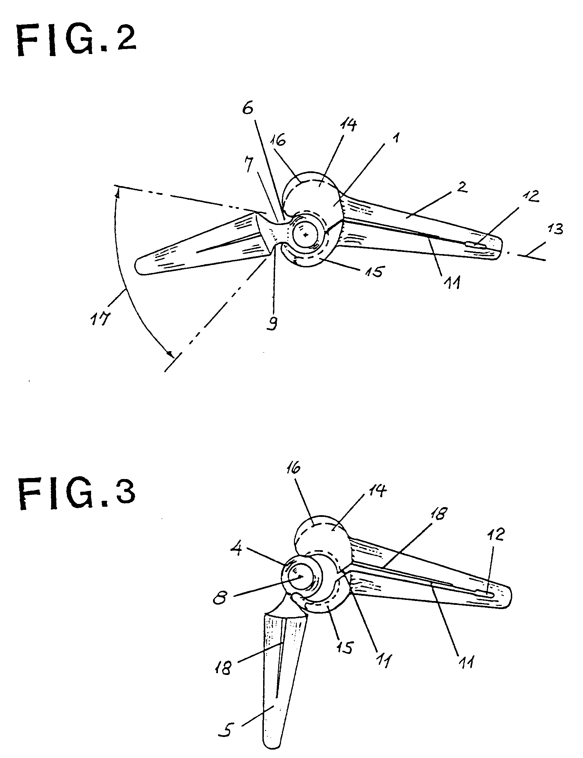

Wenn nun wie oben beschrieben der Lagerzapfen 3 definitiv im Phalanx verankert ist, wird die Lagerschale 1 mit ihrem Schaft 2 soweit in eine vorbereitete Knochenaushöhlung im Metakarpal eingeschoben, dass sie gerade noch aufspreizbar ist, um den Lagerzapfen 3 radial einzuführen. Die Längsflossen auf der oberen Hälfte 14 dienen dabei als Vorabstützung. Sie sind so bemessen, dass mit ihrem Anstehen in der vorbereiteten Knochenaushöhlung die untere Hälfte 15 noch genügend weit aufspreizbar ist. Nach dem Aufspreizen der Lagerschale 1 und dem Einführen des Lagerzapfens 3 gemäss Figur 3 kann die Lagerschale 1 mit ihrem Schaft 2 definitiv im Metakarpal eingetrieben werden, um sie mit ihrer strukturierten Oberfläche und den Längsflossen 18 zementfrei zu verankern. Im eingewachsenen Zustand ist der Flexionsbereich 17 durch Anschlagen des Halses 9 am Rand des Durchbruchs 6 begrenzt. Da die Lagerschale 1 getrennt ist, entsteht durch die Schliesskraft der federnden Hälften 14, 15 und durch die Abstützung im nicht ganz starren Knochengewebe ein weicher Anschlag. Die Lagerschale 1 besitzt auf ihrer Oberseite einen Sattelpunkt 16 zum Führen einer Strecksehne. Die nicht vom Knochengewebe umschlossenen Teile ausserhalb der Schäfte 2, 5 weisen eine polierte Oberfläche auf, um das Ansetzen von Knochengewebe zu verhindern. Die strukturierte Oberfläche der Schäfte fördert das Einwachsen. Die aufeinander gleitenden Oberflächen von Lagerschale 1, Tonne 4 und Hals 5 bestehen aus einer verschleissfesten Materialpaarung, um den Abrieb klein zu halten.

Bei einem Verzicht auf eine Schliesskraft zwischen der oberen und der unteren Hälfte der Lagerschale 1 kann der Einschnitt 11 durchgehend vorgenommen werden. In diesem Fall ist es vorteilhaft den Einschnitt 11 so zu legen, dass die voneinander vollständig getrennten Hälften 14, 15 von Lagerschale 1 und Schaft 2 im zusammengepressten Zustand in Längsrichtung 13 und in Querrichtung unverrückbar formschlüssig miteinander verbunden sind.

### Interne Stückliste

- 1: Lagerschale
- 2: Schaft (Metakarpal)
- 3: Lagerzapfen
- 4: Tonne
- 5: Schaft (Phalanx)
- 6: Durchbruch
- 7: Verbindungsstück
- 8: Achse
- 9: Hals
- 10: Ausnehmung
- 11: Einschnitt
- 12: Querdurchbruch
- 13: Längsrichtung
- 14: obere Hälfte Lagerschale
- 15: untere Hälfte Lagerschale
- 16: Sattelpunkt
- 17: Flexionsbereich
- 18: Längsflosse

## Patentansprüche

1. Fingergelenkprothese aus Metall, die aus einer Lagerschale (1) besteht, die mit einem Schaft (2) im eingesetzten Zustand im Metakarpal verankert ist und die einen Lagerzapfen (3) führt, welcher mit einem Schaft (5) im eingesetzten Zustand im Phalanx verankert ist, wobei der Lagerzapfen (3) als Tonne (4) ausgeführt ist, die quer zu ihrer Achse (8) in ihrer Mitte ein Verbindungsstück (7) zu ihrem Schaft (5) aufweist, dadurch gekennzeichnet, daß die Lagerschale (1) über ihre ganze Länge C-förmig ausgebildet und mit einem Durchbruch (6) versehen ist, dass die Lagerschale (1) die Tonne (4) mit Spiel C-förmig umschliesst, um im Rahmen dieses Spiels eine Seitenauslenkung des Phalanx-Schaftes (5) zu begrenzen und um mit dem Durchbruch (6) die Flexion des Phalanx-Schaftes zu begrenzen, und dass die Lagerschale (1) beim Einsetzen der Tonne (4) aufspreizbar ist, dadurch dass die Lagerschale (1) und ihr Schaft (2) durch einen schlitzförmigen Einschnitt (11) in Längsrichtung (13) in zwei Hälften (14, 15) geteilt sind.

2. Fingergelenkprothese nach Anspruch 1, dadurch gekennzeichnet, dass der schlitzförmige Einschnitt (11) im Schaft (2) in Längsrichtung (13) nicht durchgehend ist, sodass die zwei Hälften (14, 15) und der Schaft (2) einstückig sind und eine Biegefeder bilden, welche beim Aufspreizen der beiden Hälften (14, 15) eine Schliesskraft erzeugt.

3. Fingergelenkprothese nach Anspruch 1 oder 2, dadurch gekennzeichnet, dass das Verbindungsstück in Form eines Halses (9) ausgeführt ist, der schlanker als die angrenzenden Querschnitte von Schaft (5) und Tonne (4) ist und der in abgewinkelter Stellung in eine Ausnehmung (10) der Lagerschale (1) eintaucht.

4. Fingergelenkprothese nach einem der Ansprüche 1 bis 3, dadurch gekennzeichnet, dass die Lagerschale (1) auf der Oberseite einen Sattelpunkt (16) zum Führen einer Strecksehne aufweist.

5. Fingergelenkprothese nach einem der Ansprüche 1 bis 4, dadurch gekennzeichnet, dass Tonne (4), Verbindungsstück (7) und Lagerschale (1, 14, 15) eine polierte Oberfläche aufweisen, um das Ansetzen von Knochengewebe zu verhindern, während die Schäfte (2, 5) eine strukturierte Oberfläche aufweisen, um das Einwachsen zu fördern.

6. Fingergelenkprothese nach einem der Ansprüche 1 bis 5, dadurch gekennzeichnet, dass die aufeinandergleitenden Oberflächen von Lagerschale (1), Tonne (4) und Hals (5) aus einer verschleissfesten Paarung bestehen.

## Claims

1. A finger joint prosthesis made of metal, which consists of a bearing shell (1), which is attached to a shaft (2) in the inserted state in the metacarpal and which guides a bearing journal (3), which is attached to a shaft (5) in the inserted state in the phalange, the bearing journal (3) being designed as a barrel (4), which at right angles to its axis (8) in its centre comprises a connecting piece (7) to its shaft (5),
**characterised in that** the bearing shell (1) is constructed in a C-shape over its entire length and is provided with an opening (6),
**in that** the bearing shell (1) encloses the barrel (4) with clearance in a C-shape in order, within the limits of this clearance, to restrict a lateral excursion of the phalange shaft (5) and in order to restrict the flexion of the phalange shaft with the opening (6),
**and in that** the bearing shell (1) can be forced open when the barrel (4) is inserted by the fact that the bearing shell (1) and its shaft (2) are divided into two halves (14, 15) by a slit-shaped notch (11) in the longitudinal direction (13).

2. A finger joint prosthesis according to Claim 1,
**characterized in that** the slit-shaped notch (11) in the shaft (2) does not extend completely in the longitudinal direction (13), with the result that the two halves (14, 15) and the shaft (2) are in one piece and form a bending spring which produces a closing force when the two halves (14, 15) are forced open.

3. A finger joint prosthesis according to Claim 1 or 2, **characterized in that** the connecting piece is designed in the form of a neck (9) which is thinner than the adjacent cross sections of shaft (5) and barrel (4) and which in the bent position dips into a recess (10) in the bearing shell (1).

4. A finger joint prosthesis according to one of Claims 1 to 3,
**characterized in that** on the upper side the bearing shell (1) comprises a saddle point (16) to guide an extensor tendon.

5. A finger joint prosthesis according to one of Claims 1 to 4,
**characterized in that** barrel (4), connecting piece (7) and bearing shell (1, 14, 15) have a polished surface to prevent the deposit of osseous tissue, whereas the shafts (2, 5) comprise a structured surface so as to promote ingrowth.

6. A finger joint prosthesis according to one of Claims 1 to 5,
**characterized in that** the surfaces of bearing shell (1), barrel (4) and neck (5) sliding on one another consist of a wear-resistant combination.

## Revendications

1. Prothèse en métal d'articulation du doigt qui se compose d'une cupule (1) qui, à l'état installé, est ancrée dans un os métacarpien au moyen d'une tige (2) et qui guide un tourillon (3) qui, à l'état installé, est ancré dans une phalange au moyen d'une tige (5), le tourillon (3) étant conformé en tonneau (4) qui comporte au milieu et perpendiculairement à son axe (8) un élément (7) de raccord à sa tige (5), caractérisée en ce que la cupule (1) est conformée en C sur la totalité de sa longueur et comporte un passage (6), en ce que la cupule (1) entoure le tonneau (4) avec jeu par sa forme en C afin de limiter dans le cadre de ce jeu un pivotement latéral de la tige (5) côté phalange et afin de limiter par l'ouverture (6) la flexion de la tige côté phalange et en ce que la cupule (1) peut être écartée lors de l'introduction du tonneau (4) par le fait que la cupule (1) et sa tige (2) sont subdivisées dans la direction de la longueur (13) en deux moitiés (14, 15) par une entaille (11) en forme de fente.

2. Prothèse d'articulation du doigt selon la revendication 1, caractérisée en ce que l'entaille en forme de fente (11) ne se prolonge pas de part en part dans la tige (2) dans la direction de la longueur (13), de sorte que les deux moitiés (14, 15) et la tige (2) sont en une pièce et forment un ressort de flexion qui, lors de l'écartement des deux moitiés (14, 15), produit une force de fermeture.

3. Prothèse d'articulation du doigt selon la revendication 1 ou 2, caractérisée en ce que l'élément de raccord a la forme d'un col (9) qui est plus mince que les sections contigües de la tige (5) et du tonneau (4) et qui, lorsqu'il est en position coudée, pénètre dans un évidement (10) de la cupule (1).

4. Prothèse d'articulation du doigt selon l'une des revendications 1 à 3, caractérisée en ce que la cupule (1) comporte sur le côté supérieur un point de col (16) destiné au guidage d'un tendon d'extension.

5. Prothèse d'articulation du doigt selon l'une des revendications 1 à 4, caracterisée en ce que le tonneau (4), l'élément de raccord (7) et la cupule (1, 14, 15) ont une surface polie afin d'empêcher l'incrustation de tissu osseux, tandis que les tiges (2, 5) ont une surface structurée afin de favoriser la croissance du tissu osseux.

6. Prothèse d'articulation du doigt selon l'une des revendications 1 à 5, caractérisée en ce que les surfaces de la cupule (1), du tonneau (4) et du col (5) qui glissent les unes sur les autres sont en matériaux compatibles leur permettant de résister à l'usure.
